# EUROPÄISCHE PATENTSCHRIFT

(11) **EP 1 988 913 B1**
(45) Veröffentlichungstag und Bekanntmachung des Hinweises auf die Patenterteilung: **22.04.2009**
(21) Anmeldenummer: 07712399.0
(22) Anmeldetag: 01.03.2007
(51) Int. Cl.: A61K 38/18, A61K 9/06

(54) **G-CSF-FLÜSSIGFORMULIERUNG**
G-CSF LIQUID FORMULATION
FORMULATION LIQUIDE DE G-CSF

(30) Priorität: 01.03.2006 DE 102006009437
(43) Veröffentlichungstag der Anmeldung: 12.11.2008
(73) Patentinhaber: Bioceuticals Arzneimittel AG, 61118 Bad Vilbel (DE)
(72) Erfinder: MACK, Michael, 61118 Bad Vilbel (DE); BLASCHKE, Ulrich Kurt, 65185 Wiesbaden (DE)
(74) Vertreter: Neuefeind, Regina
(86) Internationale Anmeldenummer: PCT/EP2007/051947
(87) Internationale Veröffentlichungsnummer: WO 2007/099145

(56) Entgegenhaltungen:
- EP-A1- 0 988 861
- EP-A1- 1 060 746
- DE-A1- 10 348 550

## Beschreibung

Die vorliegende Erfindung betrifft lagerstabile Flüssigformulierungen von G-CSF und Verfahren zu deren Herstellung. Insbesondere betrifft die Erfindung Flüssigformulierungen, die G-CSF als Wirkstoff, Acetat als Puffersubstanz, Polysorbat 20 oder Polysorbat 80 als Tensid und optional pharmazeutisch annehmbare Hilfsstoffe enthalten, wobei die Formulierungen einen pH-Wert zwischen 4,1 und 4,3 aufweisen.

G-CSF (granulocyte colony stimulating factor) ist ein natürlich vorkommender Wachstumsfaktor, der zur Familie der Cytokine im weiteren Sinne und hier zur Gruppe der Kolonie-stimulierenden Faktoren gehört. G-CSF spielt eine entscheidende Rolle bei der Hämatopoese und fördert die Proliferation und Differenzierung von hämatopoetischen Vorläuferzellen und die Aktivierung von Neutrophilen. Aufgrund dieser Eigenschaften hat G-CSF Anwendung in verschiedenen medizinischen Bereichen gefunden, wie z. B. der Rekonstitution normaler Blutzellpopulationen nach der Chemotherapie oder Bestrahlung, oder zur Stimulation der Immunantwort gegenüber infektiösen Pathogenen. So wird G-CSF in der Klinik hauptsächlich bei der Tumorbekämpfung und insbesondere zur Behandlung von Neutropenie als Folge von Chemotherapie eingesetzt und findet ferner auch bei Knochenmarkstransplantationen und bei der Behandlung von Infektionskrankheiten Verwendung.

Die rekombinante Herstellung von G-CSF wurde in der Patentliteratur erstmals 1987 beschrieben, in WO-A-87/01132. Das erste kommerzielle G-CSF-Präparat auf der Basis von rekombinantem G-CSF wurde in Deutschland 1991 zugelassen und wird unter dem Handelsnamen Neupogen^{®} von der Firma Amgen hergestellt und vertrieben.

Das Produkt Neupogen^{®} (Fertigspritzen) besteht laut dem Arzneimittelverzeichnis ROTE LISTE 2005 aus folgenden Bestandteilen: G-CSF in einer Konzentration von 600 µg/ml oder 960 µg/ml, Natriumacetat, Sorbitol, Polysorbat 80 und Wasser.

Im Stand der Technik beschäftigen sich auch diverse Patentdokumente mit pharmazeutischen Zubereitungen von G-CSF. In EP-A-0 373 679 werden Formulierungen von G-CSF beschrieben, in denen das Protein durch die Anwesenheit einer Säure, einen sauren pH-Wert und eine niedrige Leitfähigkeit der Formulierung stabilisiert wird.

DE-A-37 23 781 betrifft allgemein die Verwendung von G-CSF in Kombination mit einem pharmazeutisch verträglichen grenzflächenaktiven Mittel, Saccharid, Protein oder einer pharmazeutisch verträglichen hochmolekularen Verbindung.

In WO-A-94/14465 wird die Verwendung von Maltose, Cellobiose, Gentiobiose, Isomaltose, Raffinose, Saccharose und weiterer Zucker zur Stabilisierung G-CSF-haltiger Zubereitungen beschrieben.

In WO-A-94/14466 werden G-CSF enthaltende Zubereitungen offenbart, die eine Tensidmenge, die kleiner ist als die eingesetzte Menge an G-CSF, und eine Puffersubstanz enthalten.

WO-A-93/03744 beschreibt G-CSF-haltige Formulierungen, die ein Konservierungsmittel enthalten, bei dem es sich um Chlorbutanol, Benzylalkohol oder Benzalkonium handelt.

EP-A-0 988 861 offenbart G-CSF-haltige Formulierungen, die als Puffersubstanz HEPES, TES oder Tricin enthalten.

WO-A1-2005/042024 offenbart pharmazeutische Zubereitungen von G-CSF mit einem pH-Wert von über 4,0, die eine Säure enthalten und frei von Tensiden sind.

Aufgabe der vorliegenden Erfindung ist es, eine G-CSF-Zubereitung herzustellen, die in flüssiger Form über längere Zeit gelagert werden kann und die ohne stabilisierende Zusatzstoffe wie HSA, Aminosäuren oder Konservierungsmittel auskommt. Auf diese Weise soll eine lagerstabile G-CSF-Flüssigformulierung bereitgestellt werden, die möglichst jedes Risiko für die Verträglichkeit der Formulierung vermeidet.

Diese Aufgabe wird erfindungsgemäß gelöst durch den Gegenstand des Anspruchs 1. Bevorzugte Ausführungsformen sind in den Unteransprüchen definiert.

Es wurde gefunden, dass flüssige G-CSF-Zusammensetzungen, die Acetat als Puffersubstanz und Polysorbat 20 und/oder Polysorbat 80 als Tensid enthalten und einen pH-Wert zwischen 4,1 und 4,4 aufweisen, auch bei Abwesenheit von HSA, Aminosäuren oder polymeren Stabilisierungsmitteln in flüssiger Form über längere Zeit stabil gelagert werden können; ohne dass es zu signifikanten Stabilitätsverlusten kommt.

Die Erfindung betrifft somit eine lagerstabile wässrige Flüssigformulierung von G-CSF, die neben rekombinantem humanen G-CSF als Wirkstoff Acetat als Puffer und Polysorbat 20 (Polyoxyethylen-sorbitan-monolaurat, auch als Tween 20 bezeichnet) oder Polysorbat 80 (Polyoxyethylen-sorbitan-monooleat, auch als Tween 80 bezeichnet) oder ein Gemisch davon als Tensid umfasst und einen pH-Wert zwischen 4,1 und 4,3 aufweist.

In einer bevorzugten Ausführungsform handelt es sich bei dem Tensid um Polysorbat 20.

Es wurde aber auch gefunden, dass andere Polyoxyethylensorbitanalkylester ebenfalls für den Einsatz als Detergens in den Formulierungen der vorliegenden Erfindung geeignet sind.

Das Tensid ist üblicherweise in einer Konzentration im Bereich von 0,0005 % (w/v) bis 0,05 % (w/v), bevorzugt im Bereich von 0,001 % (w/v) bis 0,01 %, besonders bevorzugt im Bereich von 0,002 % (w/v) bis 0,008 % und am meisten bevorzugt im Bereich von 0,004 % (w/v) bis 0,006 % (w/v), bezogen auf das Gesamtvolumen der Lösung, enthalten. In der Regel enthalten die erfindungsgemäßen Formulierungen das Tensid Polysorbat 20 und/oder 80 in einer Konzentration von 0,004 % (w/v), 0,005 % (w/v) oder 0,006 % (w/v).

Die Konzentration der Puffersubstanz Acetat wird vorteilhaft so gewählt, dass bei dem erfindungsgemäßen pH-Wert zwischen 4,1 und 4,3 sowohl die pHstabilisierende Wirkung als auch eine ausreichende Pufferkapazität erzielt werden, gleichzeitig aber die Ionenkonzentration und damit die Leitfähigkeit möglichst gering gehalten wird, um Aggregatbildung zu vermeiden. Üblicherweise ist Acetat in einer Konzentration im Bereich von 0,5 bis 150 mmol/l, vorzugsweise im Bereich von 1 bis 100 mmol/l, besonders bevorzugt im Bereich von 2 bis 50 mmol/l und am meisten bevorzugt zwischen 5 und 20 mmol/l enthalten. In der Regel beträgt die Konzentration des Acetats 10 mmol/l.

Die Puffersubstanz Acetat kann sowohl in Form der freien Säure als auch in Form des Salzes eingesetzt werden. Als Salze werden insbesondere die physiologisch verträglichen Salze eingesetzt, beispielsweise Alkali- oder Ammoniumsalze, bevorzugt das Natriumsalz.

Der pH-Wert der Formulierung liegt im Bereich zwischen 4,1 und 4,3 bevorzugt zwischen 4,2 und 4,3, ebenfalls besonders bevorzugt zwischen 4,25 und 4,3 und am meisten bevorzugt bei etwa 4,25 bzw. etwa 4,3.

Falls gewünscht kann der pH-Wert der Zusammensetzung auch noch mit Hilfe anderer Säuren oder Basen auf den pH im Bereich von 4,1 bis 4,3, von 4,2 bis 4,3, von 4,25 bis 4,3 bzw. von etwa 4,25 oder etwa 4,3 eingestellt werden. Geeignete Säuren sind beispielsweise Salzsäure, Phosphorsäure, Zitronensäure und Natrium- oder Kaliumhydrogenphosphat. Geeignete Basen sind beispielsweise Alkali- und Erdalkalihydroxide, Alkalicarbonate, Alkaliacetate, Alkalicitrate und Dialkalihydrogenphosphate, beispielsweise Natriumhydroxid, Natriumacetat, Natriumcarbonat, Natriumcitrat, Dinatrium- und Dikaliumhydrogenphosphat sowie Ammoniak.

Bevorzugt wird der pH mit NaOH eingestellt. Die erfindungsgemäße Formulierung enthält daher in einer Ausführungsform weiterhin Natrium-Ionen als Folge der Einstellung des pH-Wertes mit NaOH.

In einer Ausführungsform enthält die Formulierung des weiteren als pharmazeutisch annehmbaren Zusatzstoff ein Polyol, insbesondere einen Zuckeralkohol, bei dem es sich besonders bevorzugt um Mannitol oder Sorbitol handelt. Diese Zusatzstoffe eignen sich besonders gut als isotonisierende Mittel, um die erfindungsgemäßen Zusammensetzungen mit dem Blut des Patienten isotonisch zu machen.

Die Konzentration des Polyols beträgt üblicherweise bis zu 10,0 % (w/v), bezogen auf das Gesamtvolumen der Zusammensetzung. Bevorzugt beträgt die Konzentration bis zu 8,0 % (w/v), besonders bevorzugt bis zu 6,0 % (w/v). Besonders bevorzugt ist Sorbitol oder Mannitol in einer Konzentration von 5,0 % (w/v) enthalten.

Die Konzentration an G-CSF richtet sich nach der jeweils erwünschten Wirkstoffkonzentration in der Fertigspritze, in der die erfindungsgemäße Flüssigformulierung gelagert und schließlich appliziert wird. Das Handelsprodukt Neupogen^{®} (ROTE LISTE 2005, Nr. 51 038) ist bspw. in folgenden Konzentrationen erhältlich: 300 µg/0,5 ml; 480 µg/0,5 ml und 300 µg/1,0 ml. Dabei entsprechen 10 µg Protein in etwa 1,0 Mio. Intern. Einheiten (IE). Im Fall des Handelsprodukts Granocyte (ROTE LISTE Nr. 51 036) ist die Aktivität des rekombinanten G-CSF etwas höher, hier entsprechen 10 µg Protein einer Aktivität von 1,28 Mio. IE.

Typische G-CSF-Konzentrationen liegen im Rahmen der vorliegenden Erfindung zwischen 0,01 bis 3,0 mg/ml, bevorzugt zwischen 0,1 und 2,5 mg/ml, besonders bevorzugt im Bereich zwischen 0,5 und 2,0 mg/ml und am meisten bevorzugt zwischen 0,6 und 1,5 mg/ml. Die Konzentrationen 0,6 mg/ml und 0,96 mg/ml stellen bevorzugte Ausführungsformen dar. Dabei beträgt die Wirksamkeit des eingesetzten G-CSF in der Regel etwa 1,0 ± 0,6 x 10⁸ Einheiten/mg.

In höher konzentrierten Ausgangslösungen, oft als Bulk-Lösung bezeichnet, kann die Konzentration an Wirkstoff auch höher liegen, u.U. 5 mg/ml und höher.

Die erfindungsgemäßen Zusammensetzungen können weitere übliche, insbesondere physiologisch verträgliche Stabilisierungsmittel und/oder Hilfs- und Zusatzstoffe enthalten. Beispielsweise weitere Tenside, isotonierende Mittel, Reduktionsmittel, Antioxidantien, Komplexbildner, Cosolventien, Verdünnungsmittel und chaotrope Agenzien.

Allerdings wird bevorzugt, dass die Formulierung keine polymeren Stabilisierungsmittel enthält. So soll z. B. auf Polyalkylenglykole wie Polyethylenglykol, Hydroxyethylstärke, Dextrane, Cyclodextrine, aber auch auf Proteine wie HSA und andere Plasmaproteine oder Gelatine verzichtet werden.

Als Lösungsmittel wird bevorzugt reines Wasser für Injektionszwecke verwendet. Aber auch andere für pharmazeutische Zubereitungen geeignete und übliche Lösungsmittel können eingesetzt werden. Allerdings wird auf Lösungsmittel wie Glycerol, Polyethylenglykol und Propylenglykol vorzugsweise verzichtet.

Ebenfalls verzichtet wird auf den Einsatz von Puffersubstanzen wie Tartrat, Succinat, HEPES, TES und Tricin.

Auch auf Aminosäurestabilisatoren wird möglichst verzichtet.

Insgesamt ist es bevorzugt, die Anzahl der verschiedenen Hilfsstoffe in der Formulierung möglichst gering zu halten. Dementsprechend werden auch andere Zucker als Mannitol und Sorbitol möglichst vermieden, und auch bei den in der Formulierung enthaltenen Puffersubstanzen handelt es sich vorzugsweise ausschließlich um Acetat.

Die Komponenten der Formulierung können von üblichen Quellen bezogen werden, z. B. von der Firma Sigma oder der Firma Merck.

Das rekombinante G-CSF kann nach Protokollen im Stand der Technik hergestellt und gereinigt werden.

Bei dem G-CSF handelt es sich um biologisch aktives G-CSF, das in der Lage ist, die Differenzierung und Proliferation von hämatopoetischen Vorläuferzellen zu fördern und die Aktivierung von reifen Zellen des hämatopoetischen Systems zu bewirken. Die G-CSF-Formulierung eignet sich somit zur Behandlung von Indikationen, bei denen die Verabreichung von G-CSF vorteilhaft ist. Es versteht sich, dass der Begriff "biologisch aktives humanes G-CSF" auch Mutanten und Modifikationen von G-CSF einschließt, deren Aminosäuresequenz gegenüber der Wildtypsequenz verändert ist, die aber eine ähnliche biologische Aktivität wie das Wildtyp-G-CSF aufweisen, wie z. B. die in WO 01/87925 und EP 0 456 200 beschriebenen. G-CSF im Sinne der vorliegenden Erfindung sind auch G-CSF-Konjugate, in denen das Protein in konjugierter Form z. B. mit Polymeren wie etwa Polyalkylenglykolen, insbesondere mit Polyethylenglykol als sog. PEGyliertes G-CSF oder PEG-G-CSF, oder Hydroxyalkylstärken, insbesondere mit Hydroxyethylstärke, vorliegt. Das G-CSF kann glykosyliert oder nicht-glykosyliert sein. Während das in *E*. *coli* hergestellte rekombinante Protein keine Kohlenhydratstrukturen aufweist und mit einem N-terminalen Methioninrest exprimiert wird, ist das in eukaryontischen Zellen, wie bspw. CHO-Zellen, hergestellte G-CSF in der Regel glykosyliert.

Bevorzugt handelt es sich bei dem in der Flüssigformulierung enthaltenden G-CSF um humanes Met-G-CSF, hergestellt in *E. coli*-Zellen. Für die Expression in *E*. *coli*-Zellen sind verschiedene Expressionssysteme kommerziell erhältlich. Geeignet ist beispielsweise die Expression von humanem G-CSF unter der Kontrolle eines induzierbaren Promotors, beispielsweise eines IPTG-induzierbaren Promotors, siehe z. B. Sambrook and Russel, Molecular Cloning - A Laboratory Manual, 3. Auflage 2001, Coldspring Harbour Laboratory Press, Coldspring Harbour, NY, Kapitel 15, oder gängige Herstellerprotokolle, z. B. von Promega oder Stratagene.

Die Fermentation der Wirtszellen kann ebenso nach Standardprotokollen, wie sie in der Patent- und wissenschaftlichen Literatur beschrieben sind, erfolgen, wie die anschließende Aufreinigung, einschließlich der Ernte der so genannten Inclusion Bodies, die das in *E*. *coli* überexprimierte G-CSF enthalten, dem Aufschluss dieser Einschlusskörper, der Solubilisierung, Rückfaltung und chromatographischen Reinigung, für die sich geeignete Protokolle sowohl in der Patentliteratur als auch in Standardwerken der Proteinchemie sowie in Laborhandbüchern finden.

Zum Beispiel ist in EP-A-0 719 860 die Isolierung und Aufreinigung von G-CSF, einschließlich der Solubilisierung und Rückfaltung beschrieben. Allgemeine Techniken zur Solubilisierung und Renaturierung von denaturierten Proteinen sind in EP-A-0 512 097, EP-A-0 364 926, EP-A-0 219 874 und WO 01/87925 beschrieben worden und können außerdem der wissenschaftlichen Literatur und Standardwerken der Proteinchemie entnommen werden.

Das rückgefaltete Protein wird anschließend mittels chromatographischer Methoden gereinigt, d.h. es wird von anderen Proteinen und sonstigen Verunreinigungen, die nach der Solubilisierung und Renaturierung enthalten sind, getrennt.

Mit der chromatographischen Aufreinigung beschäftigt sich u.a. die bereits oben erwähnte WO 87/01132 A1, in der erstmals die Herstellung von G-CSF in *E*. *coli-*Wirtszellen beschrieben wurde. Im Rahmen der Reinigung des rekombinanten G-CSF wird in WO 87/01132 A1 in Beispiel 7 eine Kationenaustauschchromatographie unter Verwendung einer CM-Cellulose-Säule durchgeführt.

In EP 0 719 860 A1 wird das G-CSF im Anschluss an die Solubilisierung und Oxidation mittels Dowex zur Entfernung des Solubilisierungsmittels, gefolgt von einer Anionenaustauschchromatographie und einer Kationenaustauschchromatographie gereinigt. Auch in EP 0 719 860 A1 wird für die Kationenaustauschchromatographie CM-Sepharose verwendet.

In WO 03/051922 A1 wird ein Reinigungsverfahren für G-CSF beschrieben, bei dem eine Metallaffinitätschromatographie durchgeführt wird, genauer gesagt, eine Chromatographie an immobilisiertem Metall (immobilized metal affinity chromatography, IMAC). Im Anschluss an die Metallaffinitätschromatographie kann in WO 03/051922 eine Kationenaustauschchromatographie und/oder eine Gelfiltration stattfinden.

In WO 01/04154 A1 ist ein Verfahren zur Reinigung von G-CSF beschrieben, bei dem zunächst eine hydrophobe Interaktionschromatographie und darauf folgend eine Hydroxyapatit-Chromatographie durchgeführt werden. Im Anschluss an die Hydroxyapatit-Chromatographie erfolgt eine Kationenaustauschchromatographie.

Die Reinheit des G-CSF, das in die erfindungsgemäße Formulierung formuliert wird, sollte mindestens 95%, bevorzugt mindestens 97% und besonders bevorzugt mindestens 99% und am meisten bevorzugt über 99% betragen. Dabei kann die Reinheit mittels HPLC-Analysen überprüft werden. Geeignet sind hier rp-SEC- und IEX-Analysen. Geeignete Materialien und Protokolle zur Durchführung der rpHPLC oder der SEC-HPLC kann der Fachmann den Produktinformationen von Anbietern wie Vydac (http://www.vydac.com) oder TOSOH Bioscience (http://www.tosohbiosep.de) entnehmen.

In der Regel wird G-CSF in einer Reinheit von über 99%, bevorzugt über 99,5% in den erfindungsgemäßen Zubereitungen eingesetzt.

Die Wirksamkeit des eingesetzten G-CSF sollte möglichst nicht unter 50.000 IE/µg betragen, besonders geeignet ist ein G-CSF mit einer Aktivität von mindestens etwa 80.000 IE/µg, am meisten geeignet ein G-CSF mit einer Aktivität von etwa 100.000 IE/µg oder darüber.

Die Bestimmung der Ausbeute an Met-G-CSF ist auch beschrieben in Herman et al. (1996) Pharm. Biotechnol. 9: 303-328. Der genaue Anteil an Met-G-CSF wird dabei durch Integration der Peakfläche und Umrechnung anhand des Extinktionskoeffizienten ermittelt.

Nach der Aufreinigung kann das G-CSF hinsichtlich seiner Menge und seiner Aktivität analysiert werden. Eine qualitative Analyse kann über eine SDS-PAGE-Analyse mit anschließender Coomassie-Brilliant-Blue-Färbung oder eine rpHPLC erfolgen. Als Standard für die Analysen kann ein kommerziell erhältliches G-CSF-Präparat verwendet werden. Zusätzlich kann eine Peptide Map bzw. eine Massenspektroskopie durchgeführt werden. Die Aktivität des aufgereinigten G-CSF kann mit verschiedenen biologischen Testverfahren bestimmt werden, wie sie beispielsweise in Shirafuji et al. (1989) Exp. Hematol. 17(2): 116-119; Oh-Eda et al. (1990) J. Biol. Chem. 265(20): 11432-11435; Stute et al. (1992) Blood 79(11): 2849-2854 und Oshima et al. (2000) Biochem. Biophys. Res. Commun. 267(3): 924-927 beschrieben sind.

Sämtliche Chromatographien werden im Übrigen nach den Empfehlungen und Protokollen der Anbieter der Matrices bzw. der Säulen durchgeführt (z. B. bezüglich der Fließgeschwindigkeit, der zum Waschen bzw. für die Elution eingesetzten Säulenvolumen, der Durchmesser und Betthöhen der Säulen etc.).

Die biologische Aktivität des erhaltenen rekombinanten G-CSF kann durch einen Bioassay bestimmt und mit der eines Standards, kommerziell erhältlichen G-CSF (Neupogen^{®}), verglichen werden. Dazu kann die Mauszelllinie NFS-60 verwendet werden, die G-CSF-responsiv ist. Dazu wird diese Zelllinie in RPMI 1640-Medium (Firma Bachem, Heidelberg) kultiviert, das 1,5 g/l Natriumbicarbonat, 4,5 g/l Glucose, 10 mM Hepes und 1,0 mM Natriumpyruvat enthält und mit 2 mM Glutamin, 10% FCS, 0,05 mM 2-Mercaptoethanol und 60 ng/ml G-CSF supplementiert worden ist.

Für den Aktivitätstest werden die Zellen zweimal mit Medium ohne G-CSF gewaschen, in einer Konzentration von 2 x 10⁴ Zellen pro Napf in 96-Napf-Platten ausgesät und für drei Tage bei 37°C und 4,5% CO₂ mit verschiedenen Konzentrationen des gereinigten G-CSF und des Standards inkubiert. Danach werden sie mit XTT-Reagenz angefärbt und die Absorption bei 450 nm in einem Mikrotiterplattenlesegerät gemessen. Es ist erwünscht, dass die Zellen, die mit dem erhaltenen G-CSF behandelt wurden, genauso gut wachsen wie die Zellen, die mit dem Standard (z. B. das Handelsprodukt Neupogen^{®}) behandelt wurden, da dann von einer gleichen biologischen Aktivität der beiden G-CSF-Proben ausgegangen werden kann.

Die Herstellung der Formulierung erfolgt ebenfalls nach im Stand der Technik üblichen Methoden.

Üblicherweise werden die Puffer- und Tensidkomponenten und ggf. die weiteren pharmazeutisch verträglichen Hilfsstoffe zunächst in den geeigneten Mengen in dem wässrigen Lösungsmittel, gewöhnlich sterilem Wasser, gelöst. Falls erforderlich wird der pH-Wert mit Acetatlösung oder mit anderen Säuren oder Basen, wie den oben beispielhaft genannten, eingestellt. Nach einem üblichen Sterilisierungsschritt, beispielsweise Filtration durch ein Sterilfilter, wird G-CSF in der gewünschten Konzentration zugegeben. Es ist aber auch ohne weiteres möglich, G-CSF in einer wässrigen Lösung vorzulegen und den pH anschließend mit Acetat und oder geeigneten Säuren oder Basen, bevorzugt NaOH, auf den gewünschten Wert einzustellen.

Schließlich wird die fertige Flüssigformulierung in ein geeignetes Behältnis abgefüllt, in dem sie bis zur Applikation gelagert wird. Bei dem Behältnis handelt es sich insbesondere um Fertigspritzen, Durchstichflaschen oder Ampullen.

Die erfindungsmäßen Zusammensetzungen können in den verschiedenen Applikationsformen eingesetzt werden. Beispielsweise eignen sich die erfindungsgemäßen Formulierungen als Injektions- oder Infusionslösungen, insbesondere zur intravenösen, intramuskulären oder subkutanen Verabreichung. Die Zusammensetzungen können aber auch zur Herstellung weiterer Applikationsformen verwendet werden, beispielsweise von Transfersomen, Liposomen oder Hydrogelen.

Die erfindungsgemäße Flüssigformulierung bietet nicht nur die Vorteile, dass sie auf potentiell immunogene Verbindungen verzichtet und insgesamt eine möglichst geringe Anzahl von Inhaltsstoffen enthält, sondern sie erfordert auch in keiner Phase der Herstellung eine Lyophilisierung. Hierdurch werden zum einen die mit einer Lyophilisierung verbundenen Kosten eingespart, zum anderen werden die Risiken mechanischer Probleme, z. B. wenn sich das Lyophilisat nicht in vollständiger oder ausreichender Weise rekonstituieren lässt, vermieden.

Unter dem Begriff "lagerstabil" wird im Rahmen der vorliegenden Erfindung verstanden, dass der Gehalt an aktiyen G-CSF-Molekülen nach dreimonatiger Lagerung der G-CSF-Flüssigformulierung bei 25°C immer noch 80% oder mehr der Ausgangskonzentration beträgt. Vorzugsweise beträgt der Restgehalt an G-CSF-Aktivität nach dreimonatiger Lagerung bei 25°C noch mindestens 85%, bevorzugter mindestens 90% und am meisten bevorzugt mindestens 95% der Ausgangsaktivität. Die Aktivität des G-CSF kann mittels üblicher Aktivitätstests, wie sie bereits für G-CSF im Stand der Technik beschrieben sind, bestimmt werden.

Unter dem Begriff."Flüssigformulierung" wird im Rahmen der vorliegenden Erfindung verstanden, dass die Formulierung von G-CSF zusammen mit den anderen in der Formulierung enthaltenen Stoffen in keiner Phase des Herstellungsprozesses, also weder vor noch während oder nach dem Mischen der Stoffe, lyophilisiert wird und die Formulierung für die intravenöse oder subkutane Applikation als Injektions- oder Infusionslösung bestimmt ist.

In einer bevorzugten Ausführungsform enthält die Formulierung neben dem Wirkstoff G-CSF, einem Tensid ausgewählt aus Polysorbat 20, Polysorbat 80 oder einem Gemisch davon, Acetat, Sorbitol, Natrium-Ionen und Wasser keine weiteren Inhaltsstoffe und weist einen pH-Wert zwischen 4,2 und 4,3 , insbesondere zwischen 4,25 und 4,3 auf. Besonders bevorzugt ist der Einsatz von Polysorbat 20 als einziges Tensid in der Formulierung.

### Beispiele

Die folgenden Beispiele sollen die Erfindung erläutern, ohne sie einzuschränken.

G-CSF-haltige Zusammensetzungen wurden hergestellt, indem die Puffersubstanz Acetat in Form des Natriumsalzes zusammen mit Polysorbat 20 oder Polysorbat 80 und Sorbitol zunächst in destilliertem und sterilem Wasser gelöst wurden und anschließend der pH-Wert mit NaOH auf den gewünschten Wert zwischen 4,2 und 4,3 eingestellt wurde. Nicht-glykosyliertes rekominantes humanes G-CSF (Filgrastim, Met-G-CSF) wurde in der gewünschten Konzentration zugegeben. Vorzugsweise erfolgten die Herstellung und Abfüllung der Formulierung in Fertigspritzen unter Stickstoffbegasung.

Die konkreten Formulierungen der hergestellten erfindungsgemäßen Zusammensetzungen und deren pH-Werte sind in den nachfolgenden Tabellen angegeben. Bei Einsatz von Polysorbat 80 anstelle von Polysorbat 20 zeigten sich vergleichbare Ergebnisse. Gleiches gilt für die Verwendung von Mannitol anstelle von Sorbitol.

| **Bestandteil** | **Formul. 1** | **Formul. 2** | **Formul. 3** | **Formul. 4** | **Formul. 5** | **Formul. 6** |
|---|---|---|---|---|---|---|
| **G-CSF mg/ml** | 0,6 | 0,6 | 0,6 | 0,96 | 0,96 | 0,96 |
| **Sorbitol %, w/v** | 5,0 | 5,0 | 5,0 | 5,0 | 5,0 | 5,0 |
| **Acetatpuffer mM** | 10 | 10 | 10 | 10 | 10 | 10 |
| **Polysorbat20 %, w/v** | 0,004 | 0,004 | 0,004 | 0,004 | 0,004 | 0,004 |
| **NaOH 0,1 n*** | q.s. | q.s. | q.s. | q.s. | q.s. | q.s. |
| **Wasser** | 1,0 ml | ad 1,0 ml | ad 1,0 ml | ad 1,0 ml | ad 1,0 ml | ad 1,0 ml |
| **pH** | 4,2 | 4,25 | 4,3 | 4,2 | 4,25 | 4,3 |

| | | | | | | |
|---|---|---|---|---|---|---|
| * zur pH-Wert-Einstellung | | | | | | |

Die erfindungsgemäßen Formulierungen wurden zusammen mit entsprechenden Formulierungen mit einem pH-Wert von 4,0, die den Stand der Technik darstellen und als Vergleichsformulierung dienten, bei verschiedenen Temperaturen über verschiedene Zeiträume gelagert.

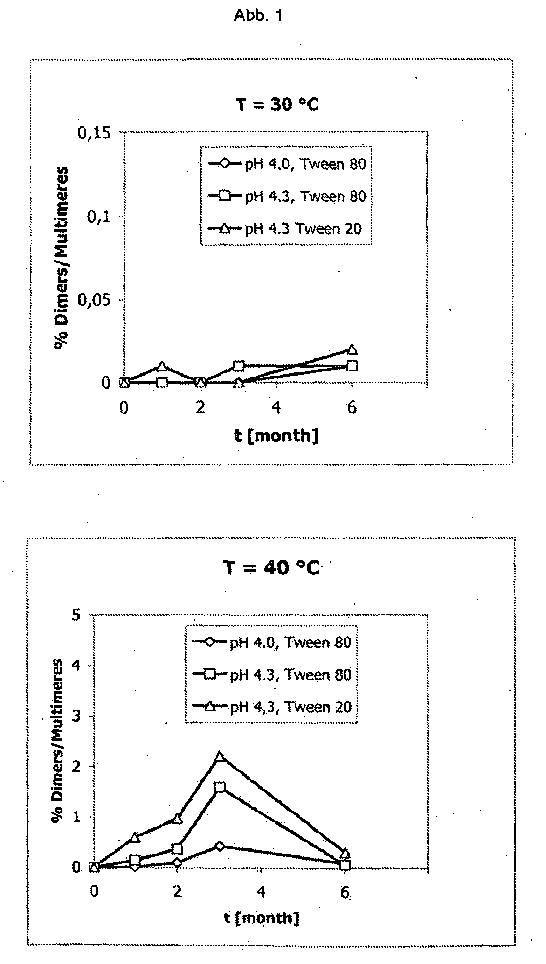

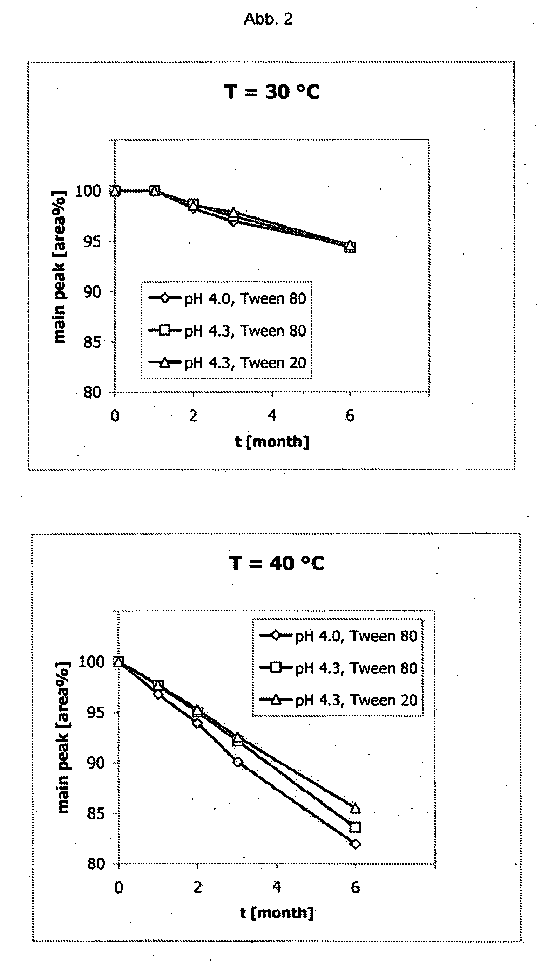

Einige Daten der Langzeitstabilitätsanalysen sind in den beigefügten Abbildungen dargestellt (G-CSF-Konzentration jeweils 0,6 mg/ml). Abbildung 1 zeigt SEC-Analysen und Abbildung 2 IEX-Analysen, jeweils bei bis zu 6-monatiger Lagerung bei 30°C bzw. 40°C.

Insgesamt zeigten die erfindungsgemäßen Formulierungen vergleichbare Ergebnisse wie die Vergleichsformulierung, die sich lediglich durch einen saureren pH-Wert, nämlich pH 4,0, von den getesteten Formulierungen gemäß der Erfindung unterschied.

## Patentansprüche

1. Flüssigformulierung von G-CSF, enthaltend G-CSF als Wirkstoff, Acetat als Puffer, Polysorbat 20 und/oder Polysorbat 80 als Tensid und optional pharmazeutisch annehmbare Hilfsstoffe, wobei die Formulierung einen pH-Wert zwischen 4,1 und 4,3 aufweist.

2. Flüssigformulierung nach Anspruch 1 mit einem pH-Wert im Bereich von 4,2 bis 4,3.

3. Formulierung nach Anspruch 1 oder 2 mit einem pH-Wert von 4,25.

4. Formulierung nach einem der vorangehenden Ansprüche, wobei die Konzentration des Acetat-Puffers zwischen 2 und 50 mmol/l beträgt.

5. Formulierung nach einem der vorangehenden Ansprüche, wobei die Konzentration des Acetat-Puffers 10 mmol/l beträgt.

6. Formulierung nach einem der vorangehenden Ansprüche, wobei die Formulierung als pharmazeutisch annehmbaren Hilfsstoff Sorbitol und/oder Mannitol enthält.

7. Formulierung nach Anspruch 6, wobei die Formulierung Sorbitol enthält.

8. Formulierung nach einem der vorangehenden Ansprüche, wobei die Formulierung kein Konservierungsmittel enthält.

9. Formulierung nach einem der vorangehenden Ansprüche, wobei die Formulierung keine Aminosäuren enthält.

10. Formulierung nach einem der vorangehenden Ansprüche, wobei die Formulierung auf polymere Stabilisierungsmittel verzichtet.

11. Formulierung nach einem der vorangehenden Ansprüche, wobei der pH mit NaOH eingestellt wurde.

12. Formulierung nach einem der vorangehenden Ansprüche, enthaltend Natrium-Ionen.

13. Formulierung nach einem der vorangehenden Ansprüche, enthaltend G-CSF, Polysorbat 20 und/oder Polysorbat 80, Sorbitol, Acetat und Natrium und darüber hinaus keine weiteren Inhaltsstoffe.

14. Formulierung nach einem der Ansprüche 1 bis 12, wobei es sich bei dem Tensid ausschließlich um Polysorbat 20 handelt.

15. Verfahren zur Herstellung einer Flüssigformulierung von G-CSF nach einem der vorangehenden Ansprüche, umfassend das Mischen des G-CSF-Proteins mit einer Lösung umfassend Acetat als Puffer, Polysorbat 20 und/oder Polysorbat 80 als Tensid und optional pharmazeutisch annehmbare Hilfsstoffe.

## Claims

1. Liquid formulation of G-CSF, containing G-CSF as active ingredient, acetate as buffer, Polysorbat 20 and/or Polysorbat 80 as surfactant and optionally pharmaceutically acceptable excipients, wherein the formulation has a pH value in the range of between 4.1 to 4.3.

2. The liquid formulation according to claim 1 having a pH value in the range of between 4.2 to 4.3.

3. Formulation according to claim 1 or 2 having a pH value of 4.25.

4. Formulation according to anyone of the preceding claims wherein the concentration of acetate buffer is between 2 and 50 mmol/l.

5. Formulation according to anyone of the preceding claims wherein the concentration of acetate buffer is 10 mmol/l.

6. Formulation according to anyone of the preceding claims wherein the formulation contains sorbitol and/or mannitol as a pharmaceutically acceptable excipient.

7. Formulation according to claim 6, wherein the formulation contains sorbitol.

8. Formulation according to anyone of the preceding claims, wherein the formulation does not contain a preservative.

9. Formulation according to anyone of the preceding claims, wherein the formulation does not contain amino acids.

10. Formulation according to anyone of the preceding claims, wherein the formulation does not contain polymeric stabilizing agents.

11. Formulation according to anyone of the preceding claims, wherein the pH was adjusted with NaOH.

12. Formulation according to anyone of the preceding claims, containing sodium ions.

13. Formulation according to anyone of the preceding claims, containing G-CSF, Polysorbat 20 and/or Polysorbat 80, sorbitol, acetate and sodium and no other ingredients.

14. Formulation according to claims 1 to 12, wherein the surfactant is exclusively Polysorbat 20.

15. Method for the production of a liquid formulation of G-CSF according to any of the preceding claims, comprising mixing of the G-CSF protein with a solution comprising acetate as buffer, Polysorbat 20 and/or Polysorbat 80 as surfactant and optionally pharmaceutically acceptable excipients.

## Revendications

1. Formulation liquide de G-CSF contenant du G-CSF comme principe actif, d'acétate comme tampon, du polysorbat 20 et/ou du polysorbat 80 comme tensio-actif et en cas échéant des adjuvants pharmaceutiquement acceptables, la formulation ayant un pH entre 4,1 et 4,3.

2. Formulation liquide selon la revendication 1 ayant un pH entre 4,2 et 4,3.

3. Formulation selon la revendication 1 ou 2 ayant un pH de 4,25.

4. Formulation selon l'une des revendications précédentes, dans laquelle la concentration du tampon d'acétate est comprise entre 2 à 50 mmol/l.

5. Formulation selon l'une des revendications précédentes, dans laquelle la concentration du tampon d'acétate est 10 mmol/l.

6. Formulation selon l'une des revendications précédentes laquelle formulation contentant comme adjuvant pharmaceutiquement acceptable du sorbitol et/ou du mannitol.

7. Formulation selon la revendication 6 laquelle formulation contentant du sorbitol.

8. Formulation selon l'une des revendications précédentes laquelle formulation ne contentant pas d'agent conservateur.

9. Formulation selon l'une des revendications précédentes laquelle formulation ne contentant pas des acides aminés.

10. Formulation selon l'une des revendications précédentes laquelle formulation est depourvue d'agents stabilisateurs polymères.

11. Formulation selon l'une des revendications précédentes, dans laquelle le pH est ajusté avec NaOH.

12. Formulation selon l'une des revendications précédentes contenant des ions de sodium.

13. Formulation selon l'une des revendications précédentes contenant du G-CSF, du polysorbat 20 et/ou du polysorbat 80, sorbitol, d'acétate et du sodium et de plus pas d'ingrédients d'avantage.

14. Formulation selon l'une des revendications 1 à 12, le tensio-actif etant exclusivement polysorbat 20.

15. Procédé de préparation d'une formulation de G-CSF selon l'une des revendications précédentes comprenant l'étape de mélanger le protéine G-CSF et une solution comprenant d'acétate comme tampon, du polysorbat 20 et/ou du polysorbat 80 comme tensio-actif et en cas échéant des adjuvants pharmaceutiquement acceptables.
